# EUROPEAN PATENT SPECIFICATION

(11) **EP 3 624 760 B1**
(45) Date of publication and mention of the grant of the patent: **27.12.2023**
(21) Application number: 18730572.7
(22) Date of filing: 18.05.2018
(51) Int. Cl.: A61K 8/34, A61K 8/37, A61K 8/41, A61K 8/42, A61Q 5/12

(54) **HAIR CARE COMPOSITION COMPRISING TRIESTER COMPOUNDS AND FATTY ESTERS OF BENZOIC ACID**
HAARPFLEGEZUSAMMENSETZUNG MIT TRIESTERVERBINDUNGEN UND FETTSÄUREESTERN VON BENZOESÄURE
COMPOSITION DE SOIN CAPILLAIRE COMPRENANT DES COMPOSÉS TRIESTER ET DES ESTERS GRAS D'ACIDE BENZOÏQUE

(30) Priority: 19.05.2017 US 201762508396 P
(43) Date of publication of application: 25.03.2020
(73) Proprietor: The Procter & Gamble Company, Cincinnati, OH 45202 (US)
(72) Inventor: SOH, MuiSiang, Singapore 138547 (SG); LIM, TianYong, Singapore 138547 (SG)
(74) Representative: P&G Patent Germany
(86) International application number: PCT/US2018/033291
(87) International publication number: WO 2018/213651

(56) References cited:
- WO-A1-99/13838
- US-A- 4 323 693
- DATABASE WPI Week 201439 Thomson Scientific, London, GB; AN 2014-L57548 XP002783503, & CN 103 735 428 A (GUANGZHOU BAIYUN DARONG FINE CHEM CO LTD) 23 April 2014 (2014-04-23) cited in the application
- Hans-Martin Haake ET AL: "Determination of the substantivity of emollients to human hair", Journal of cosmetic science, 1 July 2007 (2007-07-01), pages 443-450, XP055496541, United States Retrieved from the Internet: URL:http://www.nononsensecosmethic.org/wp- content/uploads/2015/05/Determination-of-t he-substantivity-of-emollients-to-human-ha ir.pdf [retrieved on 2018-08-01]

## Description

### FIELD OF THE INVENTION

The present invention relates to a hair care composition as defined in claim 1 comprising: a cationic surfactant system; a high melting point fatty compound; a fatty ester of benzoic acid; a triester compound; and an aqueous carrier. The compositions of the present invention provide conditioning benefits, especially dry conditioning benefits, while providing improved clean feel.

### BACKGROUND OF THE INVENTION

A variety of approaches have been developed to condition the hair. A common method of providing conditioning benefit is through the use of conditioning agents such as cationic surfactants and polymers, high melting point fatty compounds, low melting point oils, silicone compounds, and mixtures thereof. Most of these conditioning agents are known to provide various conditioning benefits.

For example, Chinese Patent Application Publication No. 103735428 A discloses a keratin conditioner which is said to be useful for restoring hair smoothness and hair damage, and discloses a composition comprising: 4-7 wt.% cetearyl alcohol, 1-2 wt.% behenyl trimethyl ammonium chloride, 1-3 wt.% cetyl trimethyl ammonium chloride, 0.5-1 wt.% 12-15C benzoate, 1-3 wt.% cyclopentasiloxane, 0.5-1.5 wt.% dimethyl siloxane, 0.5-1 wt.% diamino siloxane.

Another example is WO2013037752 which discloses a foaming oil composition comprising 50-90% of an oil component and 10-50% of a surfactant, wherein the oil component preferably comprises an ester oil with or without coconut oil In this WO publication, the composition is said to be capable of cleaning and conditioning the hair and avoid leaving the hair feeling so greasy. This WO publication also discloses 8-20C alkyl benzoate as one of the ester oils.

Furthermore, US20070071708 discloses an anhydrous composition useful for the treatment of keratinous substrate such as hair, the composition comprising at least one alkoxylated alkyl phosphate ester, at least one ester oil and optionally at least one non-ester oil component. In this US publication, the composition is said to provide exceptional cosmetic feel and improved product distribution and rinseability, product spreadability. This US publication also discloses a composition comprising 47.70% of caprylic/capric triglyceride, and 2% of 12-15C alkyl benzoate.

WO 99/13838 A1 discloses the use of a composition comprising a specific ester oil, in particular a trimethylol ester oil; a hydrophobically substituted cationic surfactant; a high melting point compound; and an aqueous carrier to provide smooth, long lasting moisturizing and non-greasy feel as well as manageability control to the hair.

US 4 323 693 A teaches that benzoic acid ester of isostearyl alcohol may be used as a non-greasy emollient in e.g. hair creams or brillantines, and that it provides improved gloss and manageability to hair.

There still exists a need for such hair care compositions, to provide conditioning benefits, especially dry conditioning benefits, while providing improved clean feel.

None of the existing art provides all of the advantages and benefits of the present invention.

### SUMMARY OF THE INVENTION

The present invention is directed to a hair care composition comprising by weight:
(a) from 0.1% to 10% of a cationic surfactant system;
(b) from 0.1 % to 20% of a high melting point fatty compound, wherein the high melting point fatty compound has a melting point of 25°C or higher, and is selected from the group consisting of fatty alcohols, fatty acids, and mixtures thereof, wherein the total amount of high melting point fatty compound ranges from 0.1 % to 20% by weight;
(c) from 0.1% to 15% of a fatty ester of benzoic acid, wherein the fatty ester of benzoic acid is the one having a fatty chain from 12 to 15 carbon atoms, and is straight or branched, saturated or unsaturated, having OH group or not, wherein the total amount of fatty ester of benzoic acid ranges from 0.1% to 15% by weight;
(d) from 0.1% to 15% of a triester compound, wherein the triester compound includes a glycerol triester being trioctanoin, wherein the total amount of triester compound ranges from 0.1 % to 15% by weight; and
(e) an aqueous carrier.

The compositions of the present invention provide conditioning benefits, especially dry conditioning benefits, while providing improved clean feel. Dry conditioning benefits herein are, for example, reduced hair volume as hair alignment, and/or reduced friction. Clean feel herein are, for example, reduced greasy, tacky, and/or sticky feel, reduced coated feel, and/or more free-flowing.

These and other features, aspects, and advantages of the present invention will become better understood from a reading of the following description, and appended claims.

### DETAILED DESCRIPTION OF THE INVENTION

While the specification concludes with claims particularly pointing out and distinctly claiming the invention, it is believed that the present invention will be better understood from the following description.

Herein, "comprising" means that other steps and other ingredients which do not affect the end result can be added. This term encompasses the terms "consisting of" and "consisting essentially of".

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Herein, "mixtures" is meant to include a simple combination of materials and any compounds that may result from their combination.

The term "molecular weight" or "M.Wt." as used herein refers to the weight average molecular weight unless otherwise stated.

### COMPOSITION

The composition comprises a cationic surfactant system; a high melting point fatty compound; a fatty ester of benzoic acid; a triester compound; and an aqueous carrier.

These ingredients, as well as the gel matrix formed by some of these ingredients, are explained below in detail.

The composition of the present invention is, preferably, substantially free of anionic surfactants in view of avoiding undesirable interaction with cationic surfactants and/or in view of stability of the gel matrix. In the present invention, "the composition being substantially free of anionic surfactants" means that: the composition is free of anionic surfactants; or, if the composition contains anionic surfactants, the level of such anionic surfactants is very low. In the present invention, the total level of such anionic surfactants is, if included, 1% or less, preferably 0.5% or less, more preferably 0.1% or less, still more preferably 0% by weight of the composition.

### CATIONIC SURFACTANT SYSTEM

The composition of the present invention comprises a cationic surfactant system. The cationic surfactant system can be one cationic surfactant or a mixture of two or more cationic surfactants. Preferably, the cationic surfactant system is selected from: mono-long alkyl quaternized ammonium salt; a combination of mono-long alkyl quaternized ammonium salt and di-long alkyl quaternized ammonium salt; mono-long alkyl amidoamine salt; a combination of mono-long alkyl amidoamine salt and di-long alkyl quaternized ammonium salt. More preferably, the cationic surfactant system is a mixture of mono-long alkyl quaternized ammonium salt and di-long alkyl quaternized ammonium salt.

The cationic surfactant system is included in the composition at a level by weight of from 0.1% to 10%, more preferably from about 0.5% to about 8%, still more preferably from about 0.8 % to about 5%, even more preferably from about 1.0% to about 4%.

### Mono-long alkyl quaternized ammonium salt

The monoalkyl quaternized ammonium salt cationic surfactants useful herein are those having one long alkyl chain which has from 12 to 30 carbon atoms, preferably from 16 to 24 carbon atoms, more preferably C18-22 alkyl group. The remaining groups attached to nitrogen are independently selected from an alkyl group of from 1 to about 4 carbon atoms or an alkoxy, polyoxyalkylene, alkylamido, hydroxyalkyl, aryl or alkylaryl group having up to about 4 carbon atoms.

Mono-long alkyl quaternized ammonium salts useful herein are those having the formula (I): 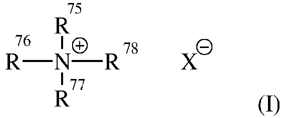 wherein one of R⁷⁵, R⁷⁶, R⁷⁷ and R⁷⁸ is selected from an alkyl group of from 12 to 30 carbon atoms or an aromatic, alkoxy, polyoxyalkylene, alkylamido, hydroxyalkyl, aryl or alkylaryl group having up to about 30 carbon atoms; the remainder of R⁷⁵, R⁷⁶, R⁷⁷ and R⁷⁸ are independently selected from an alkyl group of from 1 to about 4 carbon atoms or an alkoxy, polyoxyalkylene, alkylamido, hydroxyalkyl, aryl or alkylaryl group having up to about 4 carbon atoms; and X⁻ is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of about 12 carbons, or higher, can be saturated or unsaturated. Preferably, one of R⁷⁵, R⁷⁶, R⁷⁷ and R⁷⁸ is selected from an alkyl group of from 12 to 30 carbon atoms, more preferably from 16 to 24 carbon atoms, still more preferably from 18 to 22 carbon atoms, even more preferably 22 carbon atoms; the remainder of R⁷⁵, R⁷⁶, R⁷⁷ and R⁷⁸ are independently selected from CH₃, C₂H₅, C₂H₄OH, and mixtures thereof; and X is selected from the group consisting of Cl, Br, CH₃OSO₃, C₂H₅OSO₃, and mixtures thereof.

Nonlimiting examples of such mono-long alkyl quaternized ammonium salt cationic surfactants include: behenyl trimethyl ammonium salt; stearyl trimethyl ammonium salt; cetyl trimethyl ammonium salt; and hydrogenated tallow alkyl trimethyl ammonium salt.

### Mono-long alkyl amidoamine salt

Mono-long alkyl amines are also suitable as cationic surfactants. Primary, secondary, and tertiary fatty amines are useful. Particularly useful are tertiary amido amines having an alkyl group of from about 12 to about 22 carbons. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Useful amines in the present invention are disclosed in U.S. Patent 4,275,055, Nachtigal, et al. These amines can also be used in combination with acids such as L-glutamic acid, lactic acid, hydrochloric acid, malic acid, succinic acid, acetic acid, fumaric acid, tartaric acid, citric acid, L-glutamic hydrochloride, maleic acid, and mixtures thereof; more preferably L-glutamic acid, lactic acid, citric acid. The amines herein are preferably partially neutralized with any of the acids at a molar ratio of the amine to the acid of from about 1 : 0.3 to about 1 : 2, more preferably from about 1 : 0.4 to about 1 : 1.

### Di-long alkyl quaternized ammonium salt

Di-long alkyl quaternized ammonium salt is preferably combined with a mono-long alkyl quaternized ammonium salt or mono-long alkyl amidoamine salt. It is believed that such combination can provide easy-to rinse feel, compared to single use of a monoalkyl quaternized ammonium salt or mono-long alkyl amidoamine salt. In such combination with a mono-long alkyl quaternized ammonium salt or mono-long alkyl amidoamine salt, the di-long alkyl quaternized ammonium salts are used at a level such that the wt% of the dialkyl quaternized ammonium salt in the cationic surfactant system is in the range of preferably from about 10% to about 50%, more preferably from about 30% to about 45%.

The dialkyl quaternized ammonium salt cationic surfactants useful herein are those having two long alkyl chains having 12-30 carbon atoms, preferably 16-24 carbon atoms, more preferably 18-22 carbon atoms. The remaining groups attached to nitrogen are independently selected from an alkyl group of from 1 to about 4 carbon atoms or an alkoxy, polyoxyalkylene, alkylamido, hydroxyalkyl, aryl or alkylaryl group having up to about 4 carbon atoms.

Di-long alkyl quaternized ammonium salts useful herein are those having the formula (II): 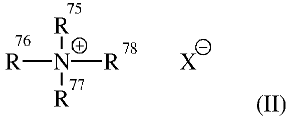 wherein two of R⁷⁵, R⁷⁶, R⁷⁷ and R⁷⁸ is selected from an alkyl group of from 12 to 30 carbon atoms or an aromatic, alkoxy, polyoxyalkylene, alkylamido, hydroxyalkyl, aryl or alkylaryl group having up to about 30 carbon atoms; the remainder of R⁷⁵, R⁷⁶, R⁷⁷ and R⁷⁸ are independently selected from an alkyl group of from 1 to about 4 carbon atoms or an alkoxy, polyoxyalkylene, alkylamido, hydroxyalkyl, aryl or alkylaryl group having up to about 4 carbon atoms; and X⁻ is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of about 12 carbons, or higher, can be saturated or unsaturated. Preferably, one of R⁷⁵, R⁷⁶, R⁷⁷ and R⁷⁸ is selected from an alkyl group of from 12 to 30 carbon atoms, more preferably from 16 to 24 carbon atoms, still more preferably from 18 to 22 carbon atoms, even more preferably 22 carbon atoms; the remainder of R⁷⁵, R⁷⁶, R⁷⁷ and R⁷⁸ are independently selected from CH₃, C₂H₅, C₂H₄OH, and mixtures thereof; and X is selected from the group consisting of Cl, Br, CH₃OSO₃, C₂H₅OSO₃, and mixtures thereof.

Such dialkyl quaternized ammonium salt cationic surfactants include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride. Such dialkyl quaternized ammonium salt cationic surfactants also include, for example, asymmetric dialkyl quaternized ammonium salt cationic surfactants.

### HIGH MELTING POINT FATTY COMPOUND

The composition of the present invention comprises a high melting point fatty compound. The high melting point fatty compound is included in the composition at a level of from 0.1% to 20%, more preferably from about 1% to about 15%, still more preferably from about 1.5% to about 8% by weight of the composition, in view of providing improved conditioning benefits such as slippery feel during the application to wet hair, softness and moisturized feel on dry hair.

The high melting point fatty compound useful herein have a melting point of 25°C or higher, and is selected from the group consisting of fatty alcohols, fatty acids, and mixtures thereof. It is understood by the artisan that, depending on the number and position of double bonds, and length and position of the branches, certain compounds having certain required carbon atoms may have a melting point of less than 25°C. Such compounds of low melting point are not included in this section. Nonlimiting examples of the high melting point compounds are found in International Cosmetic Ingredient Dictionary, Fifth Edition, 1993, and CTFA Cosmetic Ingredient Handbook, Second Edition, 1992.

Among a variety of high melting point fatty compounds, fatty alcohols are preferably used in the composition of the present invention. The fatty alcohols useful herein are those having from about 14 to about 30 carbon atoms, preferably from about 16 to about 22 carbon atoms. These fatty alcohols are saturated and can be straight or branched chain alcohols. Preferred fatty alcohols include, for example, cetyl alcohol, stearyl alcohol, behenyl alcohol, and mixtures thereof.

High melting point fatty compounds of a single compound of high purity are preferred. Single compounds of pure fatty alcohols selected from the group of pure cetyl alcohol, stearyl alcohol, and behenyl alcohol are highly preferred. By "pure" herein, what is meant is that the compound has a purity of at least about 90%, preferably at least about 95%. These single compounds of high purity provide good rinsability from the hair when the consumer rinses off the composition.

### AQUEOUS CARRIER

The conditioning composition of the present invention comprises an aqueous carrier. The level and species of the carrier are selected according to the compatibility with other components, and other desired characteristic of the product.

The carrier useful in the present invention includes water and water solutions of lower alkyl alcohols and polyhydric alcohols. The lower alkyl alcohols useful herein are monohydric alcohols having 1 to 6 carbons, more preferably ethanol and isopropanol. The polyhydric alcohols useful herein include propylene glycol, hexylene glycol, glycerin, and propane diol.

Preferably, the aqueous carrier is substantially water. Deionized water is preferably used. Water from natural sources including mineral cations can also be used, depending on the desired characteristic of the product. Generally, the compositions of the present invention comprise from about 20% to about 99%, preferably from about 30% to about 95%, and more preferably from about 80% to about 95% water.

### GEL MATRIX

The composition of the present invention comprises a gel matrix. The gel matrix comprises a cationic surfactant, a high melting point fatty compound, and an aqueous carrier.

The gel matrix is suitable for providing various conditioning benefits such as slippery feel during the application to wet hair and softness and moisturized feel on dry hair. In view of providing the above gel matrix, the cationic surfactant and the high melting point fatty compound are contained at a level such that the weight ratio of the cationic surfactant to the high melting point fatty compound is in the range of, preferably from about 1:1 to about 1:10, more preferably from about 1:1 to about 1:6.

### FATTY ESTERS OF BENZOIC ACID

The composition of the present invention comprises a fatty ester of benzoic acid.

Such fatty esters of benzoic acid are included at a level by weight of the composition of from 0.1% to 15%, more preferably from about 0.5% to about 10%, still more preferably from about 0.5% to about 8%, even more preferably from about 0.5% to about 6%, in view of providing clean feel.

Such fatty esters of benzoic acid are those having a fatty chain of from 4 12 to 15 carbon atoms, in view of providing clean feel, and can be straight or branched, saturated or unsaturated, having OH group or not. Such fatty esters of benzoic acid useful herein are preferably straight saturated alkyl benzoate without OH group.

The weight ratio of the fatty esters of benzoic acid to the triester compound is preferably from about 10:1 to 1:10, more preferably from about 8:1 to 1:8, still more preferably from about 6:1 to 1:6, in view of providing clean feel, especially reduced coated feel.

### TRIESTER COMPOUND

The composition of the present invention comprises a triester compound, wherein the triester compound includes a glycerol triester being trioctanoin.

Such triester compound is included at a level by weight of the composition of from 0.1% to 15%, more preferably from about 0.5% to about 10%, still more preferably from about 0.5% to about 8%, even more preferably from about 0.5% to about 6%. in view of providing conditioning benefits, especially dry conditioning benefits.

Preferred triester compound are those in liquid at 25°C, more preferably at 15°C, still more preferably at 11°C.

The triester compounds useful herein are those having at least three large head groups, preferably at least three hydrocarbon groups wherein each of the hydrocarbon groups has from about 5 to about 50 carbons, more preferably from about 6 to about 40 carbons, still more preferably from about 6 to about 35 carbons.

The total number of the carbon atoms of such at least three large head groups are, preferably from about 15 to about 150, more preferably from about 18 to about 120, still more preferably from about 18 to about 105.

The 3 hydrocarbon groups are independently, branched, straight, saturated, or unsaturated alkyl, aryl, and alkylaryl groups.
Such compounds useful herein are, for example, triesters. Such triesters include, for example, glycerol triesters having the following formula: 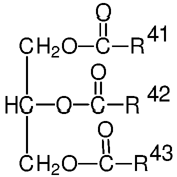 wherein R⁴¹, R⁴², and R⁴³, are explained above as three large head groups. The glycerol triesters according to the present invention include trioctanoin (also known as glycerol trioctanoate or caprylic acid triglyceride). In addition, the triesters may also include, for example, trimethylol triesters having the following formula: 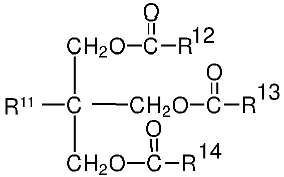 wherein R¹¹ is H, CH₃ or C₂H₅, and R¹², R¹³, and R¹⁴ are explained above as three large head groups.

Such triesters further include, for example, citrate triesters the following formula: 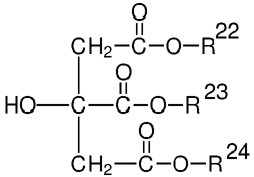 wherein R²², R²³, and R²⁴, are explained above as three large head groups.

Among the above exemplified compounds, preferred are glycerol triesters.

### SILICONE COMPOUND

The compositions of the present invention may contain silicone compounds, for example, at a level of preferably from about 0.05% to about 10%, more preferably from about 0.1% to about 5%.

Such silicones compounds can be, for example, volatile silicones such as cyclic silicones, dimethylpolysiloxane fluid, dimethylpolysiloxane gum, silicone copolyol, amino silicone with or without polyoxyalkylene groups, and quaternized aminosilicone with or without polyoxyalkylene groups.

### ADDITIONAL COMPONENTS

The composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from about 0.001% to about 10%, preferably up to about 5% by weight of the composition.

A wide variety of other additional components can be formulated into the present compositions. These include: other conditioning agents such as hydrolyzed collagen with tradename Peptein 2000 available from Hormel, vitamin E with tradename Emix-d available from Eisai, panthenol available from Roche, panthenyl ethyl ether available from Roche, hydrolyzed keratin, proteins, plant extracts, and nutrients; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents, such as any of the FD&C or D&C dyes; perfumes; and sequestering agents, such as disodium ethylenediamine tetra-acetate; and ultraviolet and infrared screening and absorbing agents such as octyl salicylate.

### PRODUCT FORMS

The conditioning compositions of the present invention can be in the form of rinse-off products or leave-on products, and can be formulated in a wide variety of product forms, including but not limited to creams, gels, emulsions, mousses and sprays.

The conditioning composition of the present invention is especially suitable for rinse-off hair conditioner. Such compositions are preferably used by following steps:
(i) after shampooing hair, applying to the hair an effective amount of the conditioning compositions for conditioning the hair; and
(ii) then rinsing the hair.

### EXAMPLES

The following examples further describe and demonstrate embodiments within the scope of the present invention. Where applicable, ingredients are identified by chemical or CTFA name, or otherwise defined below.

### Compositions (wt%)

| Components | Ex.1 | Ex.2 | Ex.3 | CEx. i | CEx. ii | CEx. iii |
|---|---|---|---|---|---|---|
| Behenyl trimethylammonium methosulfate | 3 | - | 2.2 | 3 | 3 | 3 |
| Stearamidopropyl Dimethylamine | - | 3.2 | - | - | - | - |
| Dicetyldimonium Chloride | - | - | 0.7 | - | - | - |
| L-glutamic acid | - | 1.0 | - | - | - | - |
| Cetyl alcohol | 1.2 | 4.3 | 1.2 | 1.2 | 1.2 | 1.2 |
| Stearyl alcohol | 2.9 | 3 | 3 | 2.9 | 2.9 | 2.9 |
| C12-15 alkyl benzoate | 1.5 | 1.5 | 1.5 | - | 1.5 | - |
| Triester compound (Trioctanoin) | 1.5 | 1.5 | 1.5 | - | - | 1.5 |
| Preservatives | 0.5 | 0.5 | 0.5 | 0.5 | 0.5 | 0.5 |
| Perfume | 0.6 | 0.6 | 0.6 | 0.6 | 0.6 | 0.6 |
| Deionized Water | q.s. to 100% of the composition | | | | | |
| Volume reduction | About 11.6% reduction of volume, compared to C1 | - | - | C1 | About 4.2% increase of volume, compared to C1 | About 7.1 % reduction of volume compared to C1 |

### Method of Preparation

The above hair care compositions of "Ex. 1" through "Ex. 3" and "CEx. i" through "CEx.iii" can be prepared by any conventional method well known in the art.

### Properties and Benefits

For some of the compositions, some benefits are evaluated by the following methods. Results of the evaluation are shown above in Table.

Examples 1 through 3 are hair conditioning compositions of the present invention which are particularly useful for rinse-off use. The embodiments disclosed and represented by the previous "Ex. 1" through "Ex. 3" have many advantages. For example, they provide conditioning benefits, especially dry conditioning benefits, while providing improved clean feel. Dry conditioning benefits herein are, for example, reduced hair volume as hair alignment, and/or reduced friction. Clean feel herein are, for example, reduced greasy, tacky, and/or sticky feel, reduced coated feel, and/or more free-flowing.

Such advantages especially dry conditioning can be understood by the comparison between the examples of the present invention (especially "Ex. 1") and comparative examples "CEx. i" and "CEx. iii"

### Hair volume

### (i) Preparation of hair switch

For the volume measurement, 15 gram hair switch with a length of 10inch are used. The hair switches are prepared by following steps:
(1) The hair switches are bleached and combed in the same way. Then, applying 1.5cc of non-conditioning shampoo per one hair switch, lathering, rinsing and drying the hair switches;
(2) Applying a non-conditioning shampoo at a level of 1.5cc per one hair switch and lathering the hair switch; and rinsing the hair switch;
(3) Applying conditioner at a level of 1.5cc per one hair switch and treating the hair switch;
(4) Rinsing the hair switch;
(5) Then drying the hair switch at 50°C and in low humidity (30%) environment for 1.5h; and
(6) Then placing the hair switches in a high humidity (75 %) environment, for 1h at 28 °C in which the hair switches tend to expand and increase the volume.

Hair switches are ready for volume measurements.

### (ii) Volume measurements

Volume are measured by a method described in Canadian patent application publication No. CA2567712 A1. In more detail, the volume (mm3) is measured by conducting 3D reading by the laser scanning device.

### (iii) Evaluation

The above measurement in the step (ii) are conducted on at least 3 different hair switches prepared by the step (i) per one conditioner, and then calculate an average of the volume. Reduced hair volume is considered as a result of better hair alignment.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A hair care composition comprising by weight:
(a) from 0.1% to 10% of a cationic surfactant system;
(b) from 0.1% to 20% of a high melting point fatty compound, wherein the high melting point fatty compound has a melting point of 25°C or higher, and is selected from the group consisting of fatty alcohols, fatty acids, and mixtures thereof, wherein the total amount of high melting point fatty compound ranges from 0.1% to 20% by weight;
(c) from 0.1% to 15% of a fatty ester of benzoic acid, wherein the fatty ester of benzoic acid is the one having a fatty chain from 12 to 15 carbon atoms, and is straight or branched, saturated or unsaturated, having OH group or not, wherein the total amount of fatty ester of benzoic acid ranges from 0.1% to 15% by weight;
(d) from 0.1% to 15% of a triester compound, wherein the triester compound includes a glycerol triester being trioctanoin, wherein the total amount of triester compound ranges from 0.1% to 15% by weight; and
(e) an aqueous carrier.

2. The composition of Claim 1, wherein the weight ratio of the fatty esters of benzoic acid to the triester compound is from 10:1 to 1:10, preferably from 8:1 to 1:8, more preferably from 6:1 to 1:6.

3. The composition according to claim 1, wherein the fatty ester of benzoic acid is straight saturated alkyl benzoate without OH group.

## Patentansprüche

1. Haarpflegezusammensetzung, umfassend, in Gewichtsprozent:
(a) zu 0,1 % bis 10 % ein kationenaktives Tensidsystem;
(b) zu 0,1 % bis 20 % eine Fettverbindung mit hohem Schmelzpunkt, wobei die Fettverbindung mit hohem Schmelzpunkt einen Schmelzpunkt von 25 °C oder höher aufweist und aus der Gruppe ausgewählt ist, bestehend aus Fettalkoholen, Fettsäuren und Mischungen davon, wobei die Gesamtmenge der Fettverbindung mit hohem Schmelzpunkt in einem Bereich von 0,1 Gew.-% bis 20 Gew.-% liegt;
(c) zu 0,1 % bis 15 % einen Fettsäureester der Benzoesäure, wobei der Fettsäureester der Benzoesäure der eine ist, der eine Fettkette von 12 bis 15 Kohlenstoffatomen aufweist, und geradkettig oder verzweigt, gesättigt oder ungesättigt ist, eine OH-Gruppe aufweist oder nicht, wobei die Gesamtmenge von Fettsäureester der Benzoesäure in einem Bereich von 0,1 Gew.-% bis 15 Gew.-% liegt;
(d) zu 0,1 % bis 15 % eine Triesterverbindung, wobei die Triesterverbindung einen Glycerintriester einschließt, der Trioctanoin ist, wobei die Gesamtmenge von Triesterverbindung in einem Bereich von 0,1 Gew.-% bis 15 Gew.-% liegt; und
(e) einen wässrigen Träger.

2. Zusammensetzung nach Anspruch 1, wobei das Gewichtsverhältnis von den Fettsäureestern der Benzoesäure zu der Triesterverbindung von 10 - 1 bis 1 - 10, vorzugsweise von 8 - 1 bis 1 - 8, mehr bevorzugt von 6 : 1 bis 1 : 6, beträgt.

3. Zusammensetzung nach Anspruch 1, wobei der Fettsäureester der Benzoesäure geradkettiges gesättigtes Alkylbenzoat ohne OH-Gruppe ist.

## Revendications

1. Composition pour soins capillaires comprenant en poids :
(a) de 0,1 % à 10 % d'un système tensioactif cationique ;
(b) de 0,1 % à 20 % d'un ou de plusieurs composés gras à point de fusion élevé, le composé gras à point de fusion élevé présentant un point de fusion de 25 °C ou plus, et étant choisi dans le groupe constitué par des alcools gras, des acides gras, et des mélanges de ceux-ci, la quantité totale de composés gras à point de fusion élevé allant de 0,1 % à 20 % ;
(c) de 0,1 % à 15 % d'un ester gras d'acide benzoïque, l'ester gras d'acide benzoïque étant celui présentant une chaîne grasse allant de 12 à 15 atomes de carbone, et étant linéaire ou ramifié, saturé ou insaturé, présentant un groupe OH ou non, la quantité totale d'ester gras d'acide benzoïque se stiuant dans une plage allant de 0,1 % à 15 % en poids ;
(d) de 0,1 % à 15 % d'un composé triester, le composé triester comportant un triester de glycérol étant la trioctanoïne, la quantité totale de composé triester allant de 0,1 % à 15 % en poids ; et
(e) un véhicule aqueux.

2. Composition selon la revendication 1, dans laquelle le rapport pondéral des esters gras d'acid benzoïque du composé triester va de 10:1 à 1:10, de préférence de 8:1 à 1:8, plus préférablement de 6:1 à 1:6.

3. Composition selon la revendication 1, dans laquelle l'ester gras d'acide benzoïque est le benzoate d'alkyle saturé linéaire sans groupe OH.
